Europäisches Patentamt

European Patent Office

Office européen des brevets

(19)

(11) Numéro de publication: **0 038 896**
**A1**

(12) ## DEMANDE DE BREVET EUROPEEN

(21) Numéro de dépôt: 80420050.9

(22) Date de dépôt: 28.04.80

(51) Int. Cl.³: **A 61 F 1/03**

(43) Date de publication de la demande:
04.11.81 Bulletin 81/44

(84) Etats contractants désignés:
BE CH DE GB IT LI NL

(71) Demandeur: Imbert, Jean-Claude
Les Flaches Route de St. Heand
F-42580 L'Etrat(FR)

(72) Inventeur: Imbert, Jean-Claude
Les Flaches Route de St. Heand
F-42580 L'Etrat(FR)

(74) Mandataire: Cornillon, Jacques et al,
Cabinet MAISONNIER - CORNILLON - CARADOT 15 rue
Albert Ier BP 275
F-42006 St Etienne Cedex(FR)

(54) Prothèse totale en deux parties de l'articulation fémoro-patellaire, procédé pour la mise en place de cette prothèse et jeux de gabarits pour la mise en oeuvre du procédé précité.

(57) Prothèse totale en deux parties, de l'articulation fémoro-patellaire. L'invention concerne une prothèse totale de l'articulation fémoro-patellaire réalisée en deux parties, avec un support rotulien dissymétrique.

Une pièce, comprenant une surface convexe (24) fixée dans la rotule (3) s'appuie sur une surface (28) tronc conique fixée dans le fémur (2) par une patte (29). La surface (28) est perpendiculaire à la direction de la résultante de la force d'appui (13).

Cette prothèse totale en deux parties est destinée au traitement des lésions de l'articulation fémoro-patellaire.

FIG 1

EP 0 038 896 A1

Croydon Printing Company Ltd.

TITRE MODIFIÉ
voir page de garde

– 1 –

Prothèse totale en deux parties, de l'articulation fémoro-patellaire.

La présente invention concerne une prothèse chirurgicale destinée à remédier à l'usure des cartilages de la rotule et de la Trochlée fémorale
du genou du corps humain.

On sait que le genou est constitué notamment par les articulations :

– du fémur et du tibia,

– du fémur et de la rotule.

La rotule est en continuité à sa partie supérieure avec le quadriceps
et elle est rattachée à sa partie inférieure au tibia par le tendon
rotulien. Elle est rattachée au fémur, sur les côtés inférieur et
exterieur, par des ailerons rotuliens.

Sous l'effet conjugué des différentes forces provenant du quadriceps,
du tendon rotulien et des ailerons rotuliens, la rotule est soumise à
une force d'application sur le fémur, et plus spécialement sur les
cartilages de la trochlée fémorale.

Pour cette raison, il arrive que pour certains sujets, une usure se
produise sur les cartilages de la rotule ou de la trochlée fémorale.

On connait la prothèse de MAC KEEVER, prothèse partielle, sorte de
coque métalllique destinée à recouvrir la face postérieure de la rotule et à glisser contre le cartilage trochléen.

On connait aussi les options fémoro-patellaires de la prothèse de
FREEMAN et de GUNSTON.

On connait encore la prothèse totale de BECHTOL, prothèse symétrique
avec pièce trochléenne métallique et pièce rotulienne en polyéthylène avec inclinaison invariable des deux berges.

Ces prothèses ont l'inconvénient de ne pouvoir s'adapter au cas de
l'arthrose fémoro-patellaire isolée avec subluxation rotulienne fixée.

En effet, le traitement de cette lésion comporte quatre impératifs :

1) remplacer les surfaces,

2) Recentrer la rotule par rapport au fémur,

3) S'adapter à la déformation des pièces osseuses en présence,

4) En cas d'échec, laisser un site assez peu détérioré pour qu'un rattrapage soit possible.

Si certaines prothèses connues peuvent satisfaire aux deux premiers objectifs, elles s'adaptent difficilement aux déformations parfois considérables des berges de la trochlée et, en cas d'échec, leur ablation dévoile une surface trop délabrée pour que des contraintes exercées par le tendon quadricipital après patellectomie soient bien tolérées. La présente invention a pour but de remédier à ces inconvé nients en permettant de s'adapter à la déformation des pièces osseuses.

Selon une autre caractéristique de l'invention, la prothèse est composée de deux élements :

1) Un rail trochléen en matière plastique destiné à être appliqué sur la berge usée de la trochlée fémorale,

2) Une pièce rotulienne métallique destinée à être fixée sur la face usée de la rotule en contact avec la trochlée fémorale.

Selon une autre caractéristique de l'invention, le rail trochléen en matière plastique étant appliqué sur la seule berge usée de la trochlée, et non sur les deux berges, permet ainsi de respecter l'assymétrie fonctionnelle de ladite trochlée.

Les dessins annexés, donnés à titre d'exemples non limitatifs, permettront de mieux comprendre les caractéristiques de l'invention.

La figure 1 est une vue de face d'une articulation fémur-tibia et d'une rotule, montrant les forces qui s'exercent dans le plan frontal sur celle-ci.

La figure 2 est une vue gauche de l'articulation représentée sur la figure 1, montrant les forces qui s'exercent sur la rotule dans le plan antéro-postérieur.

La figure 3 est une vue de dessus de l'articulation représentée sur la figure 2, montrant la direction de la résultante des forces s'exerçant sur une rotule.

La figure 4 est une vue d'un type d'usure de la rotule ;

La figure 5 est une vue d'un type d'usure de la trochlée.

La figure 6 est une vue de face d'une prothèse trochléenne de type connu.

La figure 7 est une vue en coupe selon AA de cette même prothèse représentée sur la figure 6.

La figure 8 est une vue en élévation de la pièce rotulienne selon l'invention.

La figure 9 est une vue de gauche de cette pièce rotulienne selon l'invention, représentée sur la figure 8.

La figure 10 est une vue de dessus de cette pièce rotulienne selon l'invention.

La figure 11 est une vue en élévation du rail trochléen, selon l'invention.

La figure 12 est une vue de gauche de ce rail selon l'invention.

La figure 13 est une vue de dessous de ce rail selon l'invention.

La figure 14 est une vue en élévation du positionnement des deux parties de la prothèse selon l'invention, en position de travail.

La figure 15 est une vue en coupe XX (figure 14) de l'ensemble de la prothèse selon l'invention, en position de travail.

La figure 16 est une vue du gabarit permettant de positionner l'encoche de fixation du rail trochléen selon l'invention.

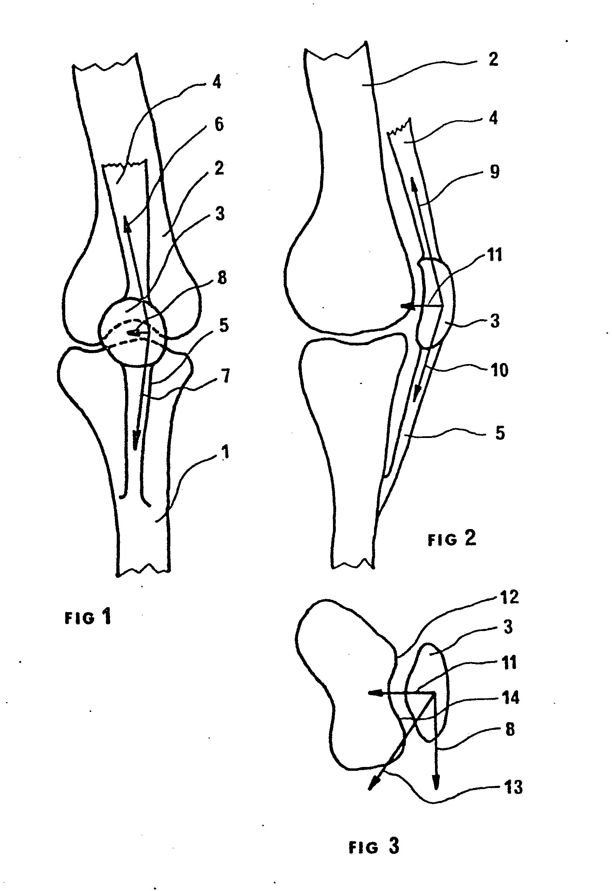

La figure 1 représente l'articulation fémoro-patellaire constituée par le tibia 1, le fémur 2 et la rotule 3.

La rotule 3 est maintenue par le quadriceps 4 et le tendon rotulien 5. La force développée par le quadriceps 4 s'exerce sur la rotule 3 selon la direction de la flèche 6.

La force développée par le tendon rotulien 5 s'exerce sur la rotule 3 selon la direction de la flèche 7.

On remarquera que les forces 6 et 7 ne sont pas directement opposées, celles-ci formant un angle très ouvert sur le côté extérieur de l'articulation, ce qui entraine une force résultante appelée force subluxante 8, appliquée sur la rotule et dirigée vers l'extérieur de l'articulation.

La figure 2 représente l'articulation fémoro-patellaire vue de côté. La quadriceps 4 exerce sur la rotule 3 une force selon la direction de la flèche 9.

Le tendon rotulien 5 exerce sur la rotule 3 une force selon la direction de la flèche 10.

On remarquera que les forces 9 et 10 ne sont pas directement opposées et forment un angle très ouvert vers l'arrière du genou, ce qui entraine une force résultante 11, appelée force d'application rotulienne s'exerçant sur la rotule 3 et dirigée contre le fémur 2.

La figure 3 représente l'articulation fémoro-patellaire vue de dessus. La rotule 3 est soumise à la force d'application rotulienne 11 dirigée contre la trochlée fémorale 12 et à la force subluxante 8 de telle sorte que la rotule 3 est, en définitive, soumise à la force résultante 13 dirigée contre une seule berge 14 de la trochlée fémorale 12.

La figure 4 représente l'articulation fémoro-patellaire vue de dessus, constituée du fémur 2 et de sa trochlée 12 sur laquelle s'articule la rotule 3. On a représenté ici le cas de l'arthrose de la rotule 3 dont le cartilage 15 a subi une importante lésion responsable d'une subluxation rotulienne fixée.

La figure 5 représente l'articulation fémoro-patellaire vue de dessus, constituée du fémur 2 et de la trochlée 12, avec ses berges 16 et 17 et, enfin, de la rotule 3.

On a représenté ici le cas de l'arthrose de la trochlée fémorale dont les cartilages de la berge 16 présentent une forte lésion responsable d'une subluxation rotulienne fixée.

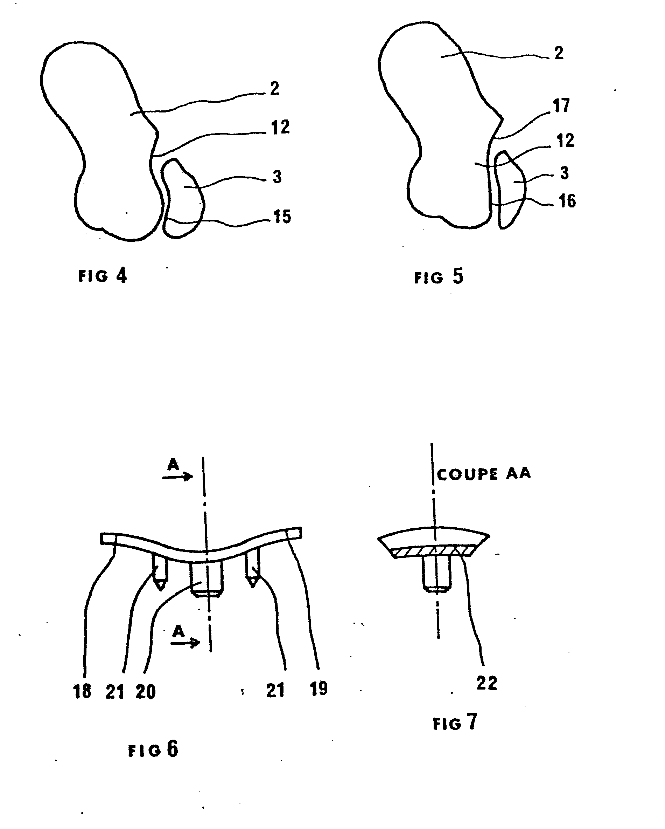

La figure 6 représente de face une prothèse trochléenne de type connu. Elle comporte deux berges 18 et 19 destinées à s'appliquer sur les berges de la trochlée fémorale, un pivot de fixation 20 destiné à s'implanter au centre de la trochlée fémorale et deux pions d'arrêt en rotation 21 destinés à s'implanter sur les berges de la trochlée.

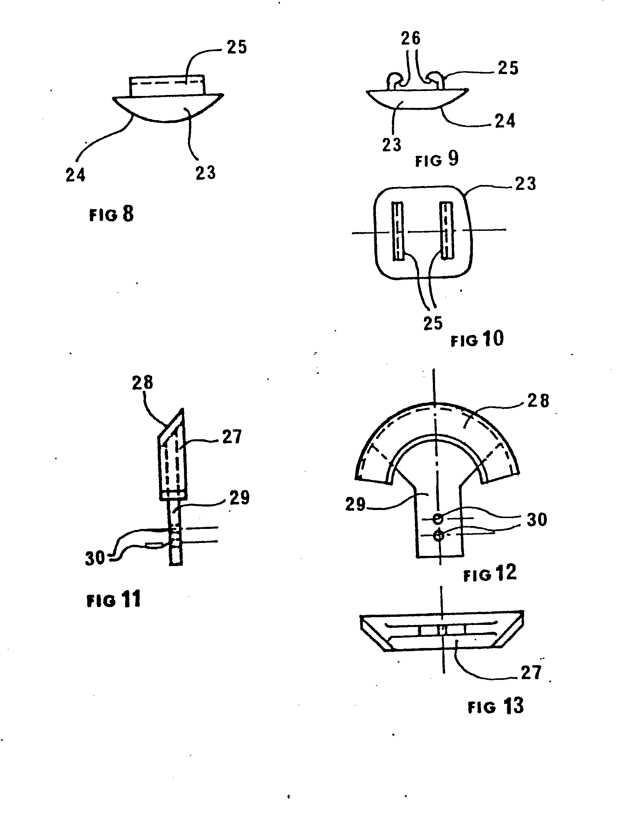

La figure 7 représente, selon la coupe AA, la prothèse trochléenne de type connu. On remarque la forme convexe de la berge 22 destinée à s'appliquer sur la berge correspondante de la trochlée fémorale.

La figure 8 représente la pièce rotulienne métallique 23 selon l'invention. Cette pièce, destinée à être fixée sur la rotule après élimination des surfaces abimées, comporte une partie convexe 24 destinée à s'appliquer sur la trochlée fémorale et des tenons 25 servant de moyens de fixation de cette pièce dans la rotule.

La figure 9 représente la pièce rotulienne selon l'invention. On a représenté les deux tenons de fixation 25. Chaque tenon comporte à son extrémité une forme en plan incliné 26 permettant un meilleur accrochage sur le ciment à l'intérieur de la cavité qui aura été préalablement pratiquée dans la rotule.

La figure 10 représente, vue de dessus, la pièce rotulienne 23 selon l'invention. On a représenté la forme extérieure de sa partie convexe inférieure, devant s'appliquer sur la trochlée fémorale.

La figure 11 est une vue en élévation du rail trochléen selon l'invention constitué à sa partie supérieure du rail proprement dit 27, comportant une inclinaison remarquable de sa face de contact 28 avec la pièce rotulienne 23 et, à sa partie inférieure, une patte de fixation 29 destinée à pénêtrer dans le fémur. Cette patte de fixation 29 comporte elle-même des trous 30 destinés à permettre au tissu osseux du fémur de pénêtrer au travers de celle-ci, ce qui renforce la solidité de la fixation du rail trochléen dans le fémur.

La figure 12 est une vue de gauche du rail trochléen selon l'invention. On remarque la forme arrondie de la surface de contact 28 permettant de s'adapter à la courbure dans le plan vertical de la trochlée fémorale. La figure 13 est une vue de dessous du rail trochléen 27 selon l'invention.

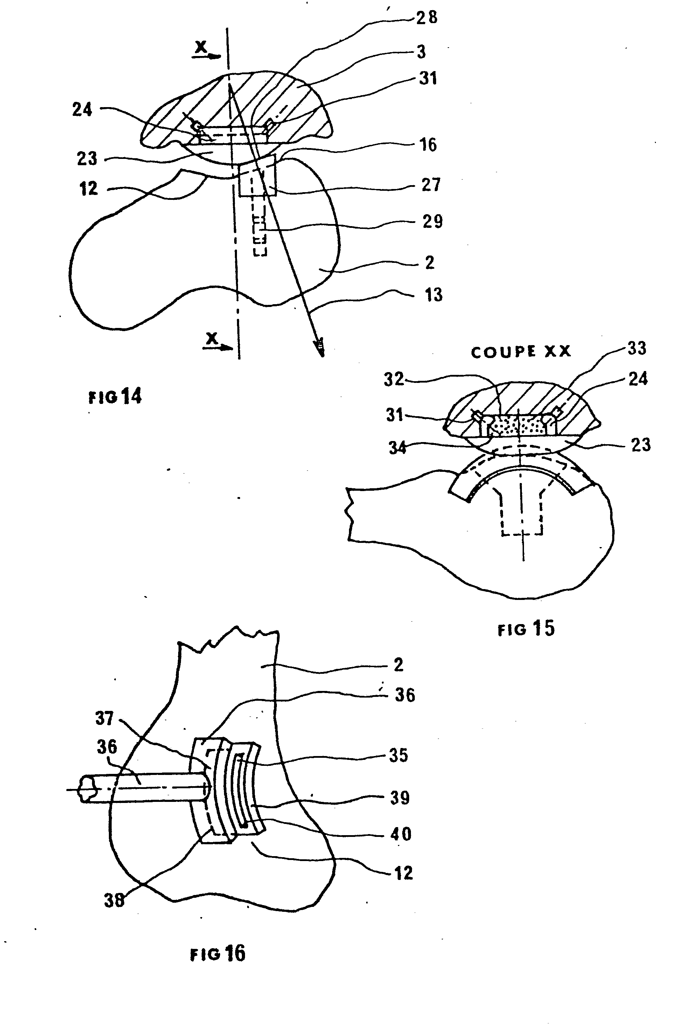

La figure 14 représente l'articulation fémoro-patellaire constituée du fémur 2 et de la rotule 3. On a placé sur la berge 16 de la trochlée fémorale 12 le rail trochléen 27 selon l'invention.

On a placé, sur la rotule 3, la pièce rotulienne 23 selon l'invention.

Le rail trochléen 27 est fixé sur la berge trochléenne 16 par la patte de fixation 29 pénêtrant dans le fémur 2.

La pièce rotulienne 23 est fixée sur la rotule 3 grâce aux tenons 25 pénétrant dans la rotule 3.

La rétention de la pièce rotulienne 23 est améliorée par l'aménagement des évidements 31 aménagés dans la rotule 3, et destinés à être garnis de ciment.

Sous l'effet du quadriceps et du tendon rotulien, la pièce rotulienne 23 est soumise à une force selon la direction de la flèche 13 dirigée contre le rail trochléen 27 et sa surface de contact 28.

On remarque l'inclinaison particulière de la surface de contact 28 du rail trochléen 27 avec la pièce rotulienne 23, permettant d'obtenir le meilleur fonctionnement possible de l'articulation, la force dirigée selon la direction de la flèche 13 étant toujours sensiblement perpendiculaire à cette surface de contact 28.

La figure 15 est une vue en coupe XX (figure 14) de la prothèse assemblée. Les tenons de fixation 25 sont placés dans une cavité parallélipipédique 32 remplie de ciment 33.

Ce ciment 33 remplit également les évidements 31, l'ensemble permettant une rétention efficace de la pièce rotulienne 23.

La face 34 de la rotule 3 a été aplanie pour un bon positionnement de la pièce rotulienne 23.

La figure 16 est une vue du fémur 2 montrant le positionnement de l'endroit où sera pratiquée l'encoche 35 de la patte de fixation du rail trochléen 27 selon l'invention.

Un gabarit 36 est appliqué sur la trochlée fémorale 12.

La surface 37 délimitée par le périmètre 38 de ce gabarit 36 est repérée. Un second gabarit 39 est placé contre le gabarit 36.

Ce gabarit 39 porte un évidement 40 correspondant à la zone de la trochlée fémorale à inciser pour recevoir la patte de fixation 29 du rail trochléen 27. La zone délimitée par le gabarit 36 sera préservée.

- 1 -

REVENDICATIONS

1. Prothèse totale en deux parties de l'articulation fémoro-patellaire constituant la surface rotulienne interne et la berge latérale support de la trochlée fémorale, caractérisée en ce qu'elle comporte une pièce de prothèse de rotule (23) à surface d'appui uniformément convexe (24) et une pièce de prothèse (27) de la trochlée fémorale à surface d'appui ayant la forme dissymétrique d'un seul secteur de surface (28) tronc conique.

2. Prothèse totale selon la revendication 1, caractérisée en ce que la pièce de prothèse patellaire (23) est symétrique et la pièce de prothèse fémorale (27) dissymétrique, implantée sur une seule berge de la trochlée fémorale.

3. Prothèse totale selon l'une quelconque des revendications précédentes, caractérisée en ce que la pièce de prothèse patellaire (23) comporte deux tenons plats parallèles (25).

4. Prothèse totale selon l'une quelconque des revendications précédentes, caractérisée en ce que chaque face en vis à vis des tenons est prolongée à son extrémité libre par des plans inclinés (26) convergents, l'espace libre entre les tenons étant plus faible à leur sommet qu'à leur base.

5. Prothèse totale selon l'une quelconque des revendications précédentes, caractérisée en ce que la pièce de prothèse fémorale comporte à l'intérieur de la surface tronc conique une patte de fixation (29) formant un angle compris entre 20 et 25° avec la génératrice de la surface tronc conique (28).

6. Prothèse totale selon l'une quelconque des revendications 1 et 4, caractérisée en ce que la patte de fixation (29) est percée d'au moins un trou (30).

7. Procédé pour la mise en place d'une prothèse totale fémoro-patellaire suivant l'une quelconque des revendications précédentes, caractérisé en ce que les tenons (25) de la pièce de prothèse patellaire (23) sont insérés dans une cavité parallélipipédique (32) dont la largeur correspond à l'espacement des faces planes extérieures des tenons, des perçages borgnes (31) étant pratiqués dans les angles de cette cavité.

8. Procédé pour la mise en place d'une prothèse fémoro-patellaire suivant l'une quelconque des revendications précédentes, caractérisé en ce que la zone de l'encoche (35) destinée à recevoir la patte de fixation de la prothèse fémorale, qui est pratiquée sur la berge extérieure de la trochlée fémorale, a sa position déterminée par un gabarit positionné sur cette trochlée.

9. Jeux de gabarits pour la mise en oeuvre du procédé suivant la revendication 7 caractérisés en ce qu'ils sont formés d'un gabarit (36) de largeur déterminée dont la surface de base est concave, et d'un gabarit (39) appliqué contre la face latérale du gabarit (36), ce gabarit portant un évidement rectangulaire (40) correspondant à la section de la patte de fixation (26) du rail trochléen (27).

**0038896**

REVENDICATIONS

1. Prothèse totale en deux parties de l'articulation fémoro-patellaire constituant la surface rotulienne interne et la berge latérale support de la trochlée fémorale, caractérisée en ce qu'elle comporte une pièce de prothèse de rotule (23) à surface d'appui uniformément convexe (24) et une pièce de prothèse (27) de la trochlée fémorale à surface d'appui ayant la forme dissymétrique d'un seul secteur de surface (28) tronc conique.

2. Prothèse totale selon la revendication 1, caractérisée en ce que la pièce de prothèse patellaire (23) est symétrique et la pièce de prothèse fémorale (27) dissymétrique, implantée sur une seule berge de la trochlée fémorale.

3. Prothèse totale selon l'une quelconque des revendications précédentes, caractérisée en ce que la pièce de prothèse patellaire (23) comporte deux tenons parallèles (25), dont chaque face en vis à vis est prolongée à son extrémité libre par un plan incliné (26), l'espace libre entre les tenons étant plus faible à leur sommet qu'à leur base.

4. Prothèse totale selon l'une quelconque des revendications précédentes, caractérisée en ce que la pièce de prothèse fémorale comporte à l'intérieur de la surface tronc conique une patte de fixation (29) formant un angle compris entre 20 et 25° avec la génératrice de la surface tronc conique (28).

5. Prothèse totale selon l'une quelconque des revendications 1 et 4, caractérisée en ce que la patte de fixation (29) est percée d'au moins un trou (30).

6. Procédé pour la mise en place d'une prothèse totale fémoro-patellaire suivant l'une quelconque des revendications précédentes, caractérisé en ce que les tenons (25) de la pièce de prothèse patellaire (23) sont insérés dans une cavité parallélipipédique (32) dont la largeur correspond à l'espacement des faces planes extérieures des tenons, des perçages borgnes (31) étant pratiqués dans les angles de cette cavité.

7. Procédé pour la mise en place d'une prothèse fémoro-patellaire suivant l'une quelconque des revendications précédentes, caractérisé en ce que la zone de l'encoche (35) destinée à recevoir la patte de fixation de la prothèse fémorale, qui est pratiquée sur la berge extérieure de la trochlée fémorale, a sa position déterminée par un gabarit positionné sur cette trochlée.

8. Jeux de gabarits pour la mise en oeuvre du procédé suivant la revendication 7, caractérisés en ce qu'ils sont formés d'un gabarit (36) de largeur déterminée dont la surface de base est concave, et d'un gabarit (39) appliqué contre la face latérale du gabarit (36), ce gabarit portant un évidement rectangulaire (40) de section identique à la section de la patte de fixation (26) du rail trochléen (27).

FIG 1

FIG 2

FIG 3

FIG 4

FIG 5

FIG 6

FIG 7

COUPE AA

FIG 8

FIG 9

FIG 10

FIG 11

FIG 12

FIG 13

FIG 14

COUPE XX

FIG 15

FIG 16

| Catégorie | Citation du document avec indication, en cas de besoin, des parties pertinentes | Revendication concernée |
|---|---|---|
| | US - A - 4 151 615 (HALL)<br>* Colonne 3, lignes 16-53 * | 1,3,5 |
| | FR - A - 2 172 104 (SULZER)<br>* Page 2, ligne 14 - page 3, ligne 35 * | 1,2,3 |
| | FR - A - 2 302 717 (PHYSICAL)<br>* Page 2, ligne 33 - page 5, ligne 35 * | 1,5 |
| | FR - A - 2 269 917 (RICHARDS)<br>* Page 3, ligne 2 - page 6, ligne 5 * | 1,3,4, 5 |
| | US - A - 4 007 495 (FRAZIER)<br>* Abrégé * | 1 |
| | FR - A - 2 403 069 (LEEDS)<br>* Page 19, lignes 19-26; figure 31 * | 8,9 |

**DOCUMENTS CONSIDERES COMME PERTINENTS**

**CLASSEMENT DE LA DEMANDE (Int. Cl. 3)**

A 61 F 1/03

**DOMAINES TECHNIQUES RECHERCHES (Int. Cl. 3)**

A 61 F

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 09-01-1981 | PESCHEK |

OEB Form 1503.1  06.78